# EUROPEAN PATENT SPECIFICATION

(11) **EP 3 395 067 B1**
(45) Date of publication and mention of the grant of the patent: **21.10.2026**
(21) Application number: 16879936.9
(22) Date of filing: 19.12.2016
(51) Int. Cl.: G06F 3/01, A61N 1/36, A61N 1/04

(54) **SYSTEM AND METHOD FOR INTEGRATING THREE DIMENSIONAL VIDEO AND GALVANIC VESTIBULAR STIMULATION**
SYSTEM UND VERFAHREN ZUR INTEGRATION VON DREIDIMENSIONALEN VIDEOSIGNALEN UND GALVANISCHER VESTIBULÄRER STIMULATION
SYSTÈME ET PROCÉDÉ PERMETTANT D'INTÉGRER LA VIDÉO 3D ET LA STIMULATION VESTIBULAIRE GALVANIQUE

(30) Priority: 23.12.2015 US 201562387586 P
(43) Date of publication of application: 31.10.2018
(73) Proprietor: Mayo Foundation for Medical Education and Research, Rochester, MN 55905 (US)
(72) Inventor: PRADHAN, Gaurav N., Fountain Hills, Arizona 85268-2013 (US); CEVETTE, Michael J., Cave Creek, Arizona 85331 (US); STEPANEK, Jan, Scottsdale, Arizona 85258 (US)
(74) Representative: Vossius & Partner Patentanwälte Rechtsanwälte mbB
(86) International application number: PCT/US2016/067560
(87) International publication number: WO 2017/112593

(56) References cited:
- JP-A- 2011 523 515
- US-A1- 2003 073 922
- US-A1- 2010 113 150
- US-A1- 2011 029 045
- US-A1- 2011 029 045
- US-A1- 2011 044 604
- US-A1- 2013 171 596
- RICHARD C. FITZPATRICK ET AL: "Probing the human vestibular system with galvanic stimulation", JOURNAL OF APPLIED PHYSIOLOGY., vol. 96, no. 6, 1 June 2004 (2004-06-01), US, pages 2301 - 2316, XP055592881, ISSN: 8750-7587, DOI: 10.1152/japplphysiol.00008.2004

## Description

### FIELD OF THE INVENTION

The invention relates generally to virtual reality stimulation systems. In particular, the invention relates to the integration of galvanic vestibular stimulation with three dimensional video.

### BACKGROUND

So-called virtual reality or enhanced reality systems such as the Rift device developed by Oculus VR present three dimensional visual stimulation to a user by displaying three dimensional video on a headset. Information representative of the orientation and/or movement of the user's head is tracked by a tracking device, and that information is used by the system to control the display of the video (e.g., so the scenes in the displayed video properly correspond to those in the direction that the user turns his or her head). It is also known to provide galvanic vestibular stimulation (GVS) in conjunction with the visual stimulation in order to provide the user with a sense of motion or acceleration to enhance the virtual experience. Systems of these types are disclosed, for example, in the Campbell U.S. Patent 5,762,612, LaValle et al. U.S. Patent 9,063,30, and the Brokken et al. U.S. Patent Application Publication 2011/0044604. GVS systems are disclosed, for example, in the Cevette et al. U.S. Patent 8,718 796. There remains, however, a continuing need for enhanced virtual reality systems. In particular, there is need for such systems that provide enhanced integrated vestibular stimulation corresponding to the visual stimulation.
US 2011/029045 A1 relates to systems and techniques for stimulating a user. For example, materials and methods for manipulating nystagmus and the related vestibular system with coupling of GVS and visual cueing are provided. Use of GVS may be applied to simulation, alleviating motion sickness, and directional cueing of a user to a precise target location.
US 2011/044604 A1 is related to an audio stream and video stream from a conventional audiovisual source which are processed by a processor. A motion processor establishes at least one motion feature and outputs it to the stimulus controller which generates a stimulus in stimulus generator. The stimulus generator may be a GVS generator.

### SUMMARY

Embodiments of the invention include a method for providing vestibular stimulation to a user corresponding to scenes of a video being viewed by the user according to claim 1, and a system configured for providing integrated three dimensional video and corresponding 3D vestibular stimulation according to claim 4.

### DESCRIPTION OF THE DRAWINGS

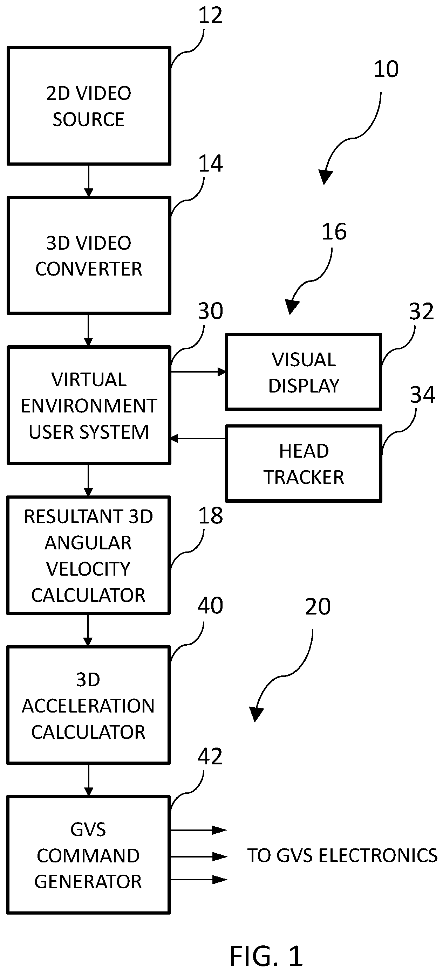

FIG. 1 is a block diagram of a three dimensional video and galvanic vestibular stimulation system in accordance with embodiments of the invention.

### DESCRIPTION OF THE INVENTION

A system 10 and method to provide integrated three dimensional (3D) video and corresponding 3D galvanic vestibular stimulation (GVS) in accordance with embodiments of the invention can be described with reference to FIG. 1. As shown, the system 10 includes two dimensional (2D) video source 12, 3D video converter 14, 3D visual stimulation system 16, resultant 3D angular velocity calculator 18 and GVS stimulation system 20. 2D video source 12 can be any desired source of a 2D video data or signal. Examples of video source 12 include a TV receiver, a DVD or other playback device, video game console, drone camera or other camera that provides 2D video signals representative of video visual scenes such as TV programming, movies, video game fields and scenes captured by a camera. In embodiments, the output of the 2D video source 12 is a composite video signal. The 2D video signal provided by the video source 12 is coupled to the 3D video converter 14. 3D video converter 14 is configured to receive a video signal over a Universal Serial Bus (USB) in embodiments of the invention. In such embodiments configured for use with a 2D video source 12 that outputs a composite video signal, a composite-to-USB converter (not shown) can be coupled between the 2D video source and the 3D video converter 14. 2D video source 12 can provide video in other formats and 3D video converter 14 can receive video in other formats in other embodiments of the invention.

3D video converter 14 converts the received 2D video signal into a corresponding 3D video data or signal. In embodiments, the 3D video converter 14 includes a computer executing Open GL (Open Graphics Library). Other embodiments are based on other 3D video formats. The 3D video signal generated by video converter 14 includes data representative of 3D video angular velocity information characteristic of the motion in the corresponding scenes of the 3D video. For example, the 3D video angular velocity information can be numerically expressed as angular velocities in each of the pitch, yaw and roll directions and represented in matrix form. In embodiments, the 3D video signal produced by the video converter 14 has a format compatible with the 3D visual stimulation system 16. Other embodiments (not shown) include a converter for converting the 3D video signal produced by the video converter 14 to a format compatible with the stimulation system 16.

3D visual stimulation system 16 includes a virtual environment user system 30, visual display 32 and head tracker 34 in the illustrated embodiment. Visual display 32 is driven by the virtual environment user system 30 and provides a 3D visual display of the 3D video received from the 3D video converter 14. In embodiments, the visual display 32 is an Oculus Rift head-mounted display of the type developed by Oculus VR. Other virtual reality display systems are used in other embodiments. Head tracker 34 generates, and provides to the virtual environment user system 30, information or signals representative of the orientation of the user's head or eyes while the user is viewing the video on the visual display 32. Head tracker 34 can, for example, be built into the visual display 32 (e.g., including accelerometers that detect motion), and/or can be external devices such as sensors. The head orientation information provided by head tracker 34 can be in the form of 3D head angular velocities representative of the user's head motion. In embodiments, the head angular velocity information provided by head tracker 34 is numerically expressed as angular velocities in each of the pitch, yaw and roll directions of the head, and is represented in matrix form. Virtual environment user system 30, which can, for example, be a video game console or a general purpose computer with video driver hardware and software, drives the visual display 32 in response to the received video signal from 3D video converter 14 and the head orientation information provided by head tracker 34 to provide the user with a visual virtual reality environment corresponding to the video and the movement of his or her head.

Resultant angular velocity calculator 18 receives the 3D video angular velocity information and the 3D head angular velocity information. In the illustrated embodiment, this video and head angular velocity information is received through the virtual environment user system 30. In other embodiments (not shown), either or both of the 3D video and head angular velocity information can be received directly from the 3D video converter 14 or the head tracker 34, respectively. Angular velocity calculator 18 calculates resultant 3D angular velocities on the basis of both the video and head motion angular velocities (i.e., the corresponding angular velocities of the user with respect to the viewed scenes of the video), and provides signals representative of that information. In embodiments, the resultant 3D angular velocities are expressed as angular velocities in each of the pitch, yaw and roll directions, and are represented in matrix form. Angular velocity calculator 18 is a programmed computer in embodiments.

GVS stimulation system 20 is coupled to receive the resultant 3D angular velocity information from the angular velocity calculator 18, and generates 3D GVS commands or signals that can be applied to the user through electrodes (not shown). The 3D GVS signals stimulate 3D acceleration sensations that correspond to the accelerations on the user viewing the video scenes. The illustrated embodiment of GVS stimulation system 20 includes 3D acceleration calculator 40 and GVS command generator 42. 3D acceleration calculator 40 processes the resultant 3D angular velocity information and calculates 3D acceleration information associated with the angular velocity information (e.g., determines the changes in the 3D angular velocities). In embodiments, the 3D acceleration information represents accelerations in each of the pitch, yaw and roll directions. 3D acceleration calculator 40 is a programmed computer in embodiments.

GVS command generator 42 converts the 3D acceleration information into electrical signals suitable and appropriate for application to the user's body (e.g., through electrodes) that will stimulate the corresponding 3D acceleration sensations in the user. In embodiments, command generator 42 generates GVS signals that provide stimulation representative of acceleration in each of the pitch, yaw and roll dimensions. In embodiments, GVS stimulation system 20 can be a four channel system that provides motion perception through four electrodes placed on the left mastoid, forehead, right mastoid, and the nape of the neck. The amplitude, locations and/or timing of the commands applied to the various electrodes can be controlled to provide GVS commands in real time in response to the 3D angular velocity information provided by angular velocity calculator 18, thereby stimulating motion perceptions corresponding to the visual stimulation presented to the user. By way of example, approaches such as those disclosed in the above-identified Cevette et al. U.S. Patent 8,718,796 can be used for this purpose. Other embodiments include other electrode numbers and placement configurations, and other stimulation approaches

In embodiments, 3D video converter 14, virtual environment user system 30, resultant 3D angular velocity calculator 18 and 3D acceleration calculator 40 can be implemented by separate computer systems or processors. In other embodiments, 3D video converter 14, virtual environment user system 30, resultant 3D angular velocity calculator 18 and 3D acceleration calculator 40 represent logical functions, and all or some or all of these functions can be performed by other computer system architectures. For example, the functions performed by 3D video converter 14, virtual environment user system 30 and resultant angular velocity calculator 18 can be provided by a GVS motion app on one computer system.

Embodiments of the invention offer important advantages. For example, they can enhance the virtual reality experience of users by providing motion stimulation that closely represents the motion corresponding to the video scene. The system and method are flexible in that they can be used in connection with any desired 2D video source. The system and method are also efficient to implement.

Although the invention has been described with reference to preferred embodiments, those skilled in the art will recognize that changes can be made in form and detail without departing from the scope of the invention. For example, as used herein, the term "virtual reality" is intended to broadly encompass any system and method that provides at least in part electronically generated stimulation to a user, including for example so-called "augmented reality" environments.

## Claims

1. A method for providing vestibular stimulation to a user corresponding to scenes of a video being viewed by the user, comprising:
receiving a three dimensional video signal including three dimensional video angular velocity information, wherein the video signal is representative of scenes of the video that can be displayed to the user, and wherein the three dimensional video angular velocity information is characteristic of motion in the corresponding scenes of the video;
displaying the scenes represented by the video to the user;
receiving a three dimensional head orientation signal including three dimensional head angular velocity information, wherein the head orientation signal is representative of the motion of the head of the user viewing the displayed scenes;
calculating resultant three dimensional angular velocity information based on the video angular velocity information and the head angular velocity information, wherein the resultant three dimensional angular velocity information is representative of three dimensional angular velocities of the user's head with respect to the viewed scenes;
driving a visual display (32) in response to the received three dimensional video signal and the received three dimensional head orientation signal to provide the user with a visual virtual reality environment corresponding to the scenes represented by the video and the movement of his or her head;
calculating three dimensional acceleration information based on the resultant three dimensional angular velocity information by determining changes in the three dimensional velocities, wherein the three dimensional acceleration information is associated with the resultant angular velocity information; and
generating three dimensional vestibular stimulation signals corresponding to the three dimensional angular acceleration information, wherein the three dimensional stimulation signals are configured to stimulate three dimensional acceleration sensations corresponding to accelerations on the user viewing the displayed scenes; and
applying the three dimensional vestibular stimulation signals to the user while displaying the scenes.

2. The method of claim 1 wherein the three dimensional stimulation signals provide stimulation representative of acceleration in each of the pitch, yaw and roll directions.

3. The method of claim 1 or 2 and further including:
receiving a two dimensional video signal; and
generating the three dimensional video signal based on the two dimensional video signal.

4. A system configured for providing integrated three dimensional, 3D, video and corresponding 3D vestibular stimulation, comprising:
a 3D visual stimulation system (16) including:
a virtual environment user system (30) providing a three dimensional video signal including three dimensional video angular velocity information, wherein the video signal is representative of scenes of a video that can be viewed by a user, and wherein the three dimensional video angular velocity information is characteristic of motion in the corresponding scenes of the video;
a visual display (32) coupled to the virtual environment user system (30) to display the scenes represented by the video to the user; and
a head tracker (34) coupled to the virtual environment user system (30) and providing a three dimensional head orientation signal including three dimensional head angular velocity information, wherein the head orientation signal is representative of the motion of the head of the user viewing the displayed scenes
wherein the visual display (32) is configured to provide the user with a visual virtual reality environment corresponding to the scenes represented by the video and the movement of his or her head in response to the three dimensional video signal and the three dimensional head orientation signal to; and
electrodes configured to apply vestibular stimulation signals to the user's body;
a 3D angular velocity calculator (18) coupled to the 3D visual stimulation system (16) and configured for calculating resultant three dimensional angular velocity information based on the video angular velocity information and the head angular velocity information, wherein the resultant three dimensional angular velocity information is representative of three dimensional angular velocities of the user's head with respect to the viewed scenes;
a 3D acceleration calculator (40) coupled to the 3D angular velocity calculator (18) and configured for calculating three dimensional acceleration information based on the resultant three dimensional angular velocity information by determining changes in the three dimensional velocities, wherein the three dimensional acceleration information is associated with the resultant angular velocity information; and
a 3D vestibular stimulation command generator (42) coupled to the 3D acceleration calculator (40) and the electrodes, the vestibular stimulation command generator generating three dimensional vestibular stimulation signals corresponding to the three dimensional angular acceleration information, wherein the three dimensional stimulation signals are applied to the electrodes and configured to stimulate three dimensional acceleration sensations corresponding to accelerations on the user viewing the displayed scenes.

5. The system of claim 4 and further including a 3D video converter (14) coupled to the 3D visual stimulation system (16) and configured for generating the three dimensional video signal based on a two dimensional video signal.

6. The system of claim 5 and further including a two dimensional video source (12) coupled to the 3D video converter (14), wherein the two dimensional video source provides the two dimensional video signal.

7. The system of claim 6 wherein the three dimensional stimulation signals provide stimulation representative of acceleration in each of the pitch, yaw and roll directions.

## Patentansprüche

1. Verfahren zum Bereitstellen einer vestibulären Stimulation für einen Benutzer, die Szenen eines Videos entspricht, das vom Benutzer betrachtet wird, das aufweist:
Empfangen eines dreidimensionalen Videosignals, das dreidimensionale Video-Winkelgeschwindigkeitsinformationen enthält, wobei das Videosignal für Szenen des Videos repräsentativ ist, die dem Benutzer angezeigt werden können, und wobei die dreidimensionalen Video-Winkelgeschwindigkeitsinformationen für eine Bewegung in den entsprechenden Szenen des Videos charakteristisch sind;
Anzeigen der durch das Video repräsentierten Szenen für den Benutzer;
Empfangen eines dreidimensionalen Kopfausrichtungssignals, das dreidimensionale Kopf-Winkelgeschwindigkeitsinformationen enthält, wobei das Kopfausrichtungssignal für die Bewegung des Kopfes des Benutzers repräsentativ ist, der die angezeigten Szenen betrachtet;
Berechnen resultierender dreidimensionaler Winkelgeschwindigkeitsinformationen basierend auf den Video-Winkelgeschwindigkeitsinformationen und den Kopf-Winkelgeschwindigkeitsinformationen, wobei die resultierenden dreidimensionalen Winkelgeschwindigkeitsinformationen für dreidimensionalen Winkelgeschwindigkeiten des Kopfes des Benutzers in Bezug auf die betrachteten Szenen repräsentativ sind;
Ansteuern einer visuellen Anzeige (32) als Reaktion auf das empfangene dreidimensionale Videosignal und das empfangene dreidimensionale Kopfausrichtungssignal, um dem Benutzer eine visuelle Virtual-Reality-Umgebung bereitzustellen, die den durch das Video repräsentierten Szenen und der Bewegung seines Kopfes entspricht;
Berechnen dreidimensionaler Beschleunigungsinformationen basierend auf den resultierenden dreidimensionalen Winkelgeschwindigkeitsinformationen durch Bestimmen von Änderungen der dreidimensionalen Geschwindigkeiten, wobei die dreidimensionalen Beschleunigungsinformationen mit den resultierenden Winkelgeschwindigkeitsinformationen assoziiert sind; und
Erzeugen dreidimensionaler vestibulärer Stimulationssignale, die den dreidimensionalen Winkelbeschleunigungsinformationen entsprechen, wobei die dreidimensionalen Stimulationssignale konfiguriert sind, dreidimensionale Beschleunigungsempfindungen zu stimulieren, die den Beschleunigungen des Benutzers entsprechen, der die angezeigten Szenen betrachtet; und
Anwenden der dreidimensionalen vestibulären Stimulationssignale auf den Benutzer während die Szenen angezeigt werden.

2. Verfahren nach Anspruch 1, wobei die dreidimensionalen Stimulationssignale eine Stimulation bereitstellen, die für die Beschleunigung in jeder der Neigungs-, Gier- und Rollrichtungen repräsentativ ist.

3. Verfahren nach Anspruch 1 oder 2, das ferner aufweist:
Empfangen eines zweidimensionalen Videosignals; und
Erzeugen des dreidimensionalen Videosignals basierend auf dem zweidimensionalen Videosignal.

4. System, das zum Bereitstellen eines integrierten dreidimensionalen, 3D, Videos und einer entsprechenden vestibulären 3D-Stimulation konfiguriert ist, das aufweist:
ein visuelles 3D Stimulationssystem (16), das aufweist:
ein Benutzersystem (30) virtueller Umgebung, das ein dreidimensionales Videosignal bereitstellt, das dreidimensionale Video-Winkelgeschwindigkeitsinformationen enthält, wobei das Videosignal für Szenen eines Videos repräsentativ ist, die von einem Benutzer betrachtet werden können, und wobei die dreidimensionalen Video-Winkelgeschwindigkeitsinformationen für eine Bewegung in den entsprechenden Szenen des Videos charakteristisch sind;
eine visuelle Anzeige (32), die mit dem Benutzersystem (30) virtueller Umgebung gekoppelt ist, um dem Benutzer die durch das Video repräsentierten Szenen anzuzeigen; und
einen Kopf-Tracker (34), der mit dem Benutzersystem (30) virtueller Umgebung gekoppelt ist und ein dreidimensionales Kopfausrichtungssignal bereitstellt, das dreidimensionale Kopf-Winkelgeschwindigkeitsinformationen enthält, wobei das Kopfausrichtungssignal die Bewegung des Kopfes des Benutzers repräsentiert, der die angezeigten Szenen betrachtet
wobei die visuelle Anzeige (32) konfiguriert ist, dem Benutzer eine visuelle Virtual-Reality-Umgebung bereitzustellen, die den durch das Video repräsentierten Szenen und der Bewegung seines Kopfes entspricht, als Reaktion auf das dreidimensionale Videosignal und das dreidimensionale Kopfausrichtungssignal; und
Elektroden, die konfiguriert sind, auf den Körper des Benutzers vestibuläre Stimulationssignale anzuwenden;
einen 3D-Winkelgeschwindigkeitsrechner (18), der mit dem visuellen 3D-Stimulationssystem (16) gekoppelt ist und zum Berechnen resultierender dreidimensionaler Winkelgeschwindigkeitsinformationen basierend auf den Video-Winkelgeschwindigkeitsinformationen und den Kopf-Winkelgeschwindigkeitsinformationen konfiguriert ist, wobei die resultierenden dreidimensionalen Winkelgeschwindigkeitsinformationen für die dreidimensionalen Winkelgeschwindigkeiten des Kopfes des Benutzers in Bezug auf die betrachteten Szenen repräsentativ sind;
einen 3D-Beschleunigungsrechner (40), der mit dem 3D-Winkelgeschwindigkeitsrechner (18) gekoppelt ist und zum Berechnen dreidimensionaler Beschleunigungsinformationen basierend auf der resultierenden dreidimensionalen Winkelgeschwindigkeitsinformationen durch Bestimmung von Änderungen der dreidimensionalen Geschwindigkeiten konfiguriert ist, wobei die dreidimensionalen Beschleunigungsinformationen mit den resultierenden Winkelgeschwindigkeitsinformationen assoziiert sind; und
einen Befehlsgenerator (42) einer vestibulären 3D-Stimulation, der mit dem 3D-Beschleunigungsrechner (40) und den Elektroden gekoppelt ist, wobei der Befehlsgenerator einer vestibulären 3D-Stimulation dreidimensionale vestibuläre Stimulationssignale erzeugt, die den dreidimensionalen Winkelbeschleunigungsinformationen entsprechen, wobei die dreidimensionalen Stimulationssignale an die Elektroden angelegt werden und konfiguriert sind, dreidimensionale Beschleunigungsempfindungen zu stimulieren, die den Beschleunigungen des Benutzers entsprechen, der die angezeigten Szenen betrachtet.

5. System nach Anspruch 4, das ferner einen 3D-Videokonverter (14) aufweist, der mit dem visuellen 3D Stimulationssystem (16) gekoppelt ist und zum Erzeugen des dreidimensionalen Videosignals basierend auf einem zweidimensionalen Videosignal konfiguriert ist.

6. System nach Anspruch 5, das ferner eine zweidimensionale Videoquelle (12) aufweist, die mit dem 3D-Videokonverter (14) gekoppelt ist, wobei die zweidimensionale Videoquelle das zweidimensionale Videosignal bereitstellt.

7. System nach Anspruch 6, wobei die dreidimensionalen Stimulationssignale eine Stimulation bereitstellen, die für eine Beschleunigung in jeder der Neigungs-, Gier- und Rollrichtungen repräsentativ ist.

## Revendications

1. Procédé permettant de fournir à un utilisateur une stimulation vestibulaire correspondant aux scènes d'une vidéo qu'il est en train de visionner, comprenant :
la réception d'un signal vidéo tridimensionnel comprenant des informations de vitesse angulaire vidéo tridimensionnelle, le signal vidéo étant représentatif de scènes de la vidéo pouvant être affichées à l'utilisateur, et les informations de vitesse angulaire vidéo tridimensionnelle étant caractéristiques d'un mouvement dans les scènes correspondantes de la vidéo ;
l'affichage à l'utilisateur des scènes représentées par la vidéo ;
la réception d'un signal d'orientation tridimensionnelle de la tête comprenant des informations de vitesse angulaire tridimensionnelle de la tête, le signal d'orientation de la tête étant représentatif du mouvement de la tête de l'utilisateur visionnant les scènes affichées ;
le calcul des informations de vitesse angulaire tridimensionnelle résultantes sur la base des informations de vitesse angulaire vidéo et des informations de vitesse angulaire de la tête, les informations de vitesse angulaire tridimensionnelle résultantes représentant les vitesses angulaires tridimensionnelles de la tête de l'utilisateur par rapport aux scènes visionnées ;
la commande d'un écran d'affichage (32) en réponse au signal vidéo tridimensionnel reçu et au signal d'orientation tridimensionnelle de la tête reçu afin de fournir à l'utilisateur un environnement visuel de réalité virtuelle correspondant aux scènes représentées par la vidéo et au mouvement de sa tête ;
le calcul des informations d'accélération tridimensionnelle à partir des informations de vitesse angulaire tridimensionnelle résultantes en déterminant les variations des vitesses tridimensionnelles, les informations d'accélération tridimensionnelles étant associées aux informations de vitesse angulaire résultantes ; et
la génération des signaux de stimulation vestibulaire tridimensionnelle correspondant aux informations d'accélération angulaire tridimensionnelle, les signaux de stimulation tridimensionnelle étant configurés pour stimuler des sensations d'accélération tridimensionnelle correspondant aux accélérations subies par l'utilisateur qui visualise les scènes affichées ; et
l'application des signaux de stimulation vestibulaire tridimensionnelle à l'utilisateur tout en affichant les scènes.

2. Procédé selon la revendication 1, dans lequel les signaux de stimulation tridimensionnelle fournissent une stimulation représentative de l'accélération dans chacune des directions de tangage, de lacet et de roulis.

3. Procédé selon la revendication 1 ou 2, comprenant en outre :
la réception d'un signal vidéo bidimensionnel ; et
la génération du signal vidéo tridimensionnel à partir du signal vidéo bidimensionnel.

4. Système configuré pour fournir une vidéo tridimensionnelle 3D intégrée et une stimulation vestibulaire 3D correspondante, comprenant :
un système de stimulation visuelle 3D (16) comprenant :
un système utilisateur d'environnement virtuel (30) fournissant un signal vidéo tridimensionnel comprenant des informations de vitesse angulaire vidéo tridimensionnelle, dans lequel le signal vidéo est représentatif de scènes d'une vidéo pouvant être visionnées par un utilisateur, et dans lequel les informations de vitesse angulaire vidéo tridimensionnelle sont caractéristiques d'un mouvement dans les scènes correspondantes de la vidéo ;
un écran d'affichage (32) couplé au système utilisateur d'environnement virtuel (30) pour afficher à l'utilisateur les scènes représentées par la vidéo ; et
un dispositif de suivi de la tête (34) couplé au système utilisateur d'environnement virtuel (30) et fournissant un signal d'orientation tridimensionnelle de la tête comprenant des informations de vitesse angulaire tridimensionnelle de la tête, le signal d'orientation de la tête étant représentatif du mouvement de la tête de l'utilisateur visionnant les scènes affichées
dans lequel l'écran d'affichage (32) est configuré pour fournir à l'utilisateur un environnement visuel de réalité virtuelle correspondant aux scènes représentées par la vidéo et au mouvement de sa tête en réponse au signal vidéo tridimensionnel et au signal d'orientation tridimensionnelle de la tête ; et
des électrodes configurées pour appliquer des signaux de stimulation vestibulaire au corps de l'utilisateur ;
un calculateur de vitesse angulaire 3D (18) couplé au système de stimulation visuelle 3D (16) et configuré pour calculer des informations de vitesse angulaire tridimensionnelle résultantes sur la base des informations de vitesse angulaire vidéo et des informations de vitesse angulaire de la tête, les informations de vitesse angulaire tridimensionnelle résultantes étant représentatives des vitesses angulaires tridimensionnelles de la tête de l'utilisateur par rapport aux scènes visionnées ;
un calculateur d'accélération 3D (40) couplé au calculateur de vitesse angulaire 3D (18) et configuré pour calculer des informations d'accélération tridimensionnelles sur la base des informations de vitesse angulaire tridimensionnelle résultantes en déterminant les variations des vitesses tridimensionnelles, les informations d'accélération tridimensionnelles étant associées aux informations de vitesse angulaire résultantes ; et
un générateur de commandes de stimulation vestibulaire 3D (42) couplé au calculateur d'accélération 3D (40) et aux électrodes, le générateur de commandes de stimulation vestibulaire générant des signaux de stimulation vestibulaire tridimensionnelle correspondant aux informations d'accélération angulaire tridimensionnelle, les signaux de stimulation tridimensionnels étant appliqués aux électrodes et configurés pour stimuler des sensations d'accélération tridimensionnelle correspondant aux accélérations subies par l'utilisateur qui visualise les scènes affichées.

5. Système selon la revendication 4, comprenant en outre un convertisseur vidéo 3D (14) couplé au système de stimulation visuelle 3D (16) et configuré pour générer le signal vidéo tridimensionnel à partir d'un signal vidéo bidimensionnel.

6. Système selon la revendication 5, comprenant en outre une source vidéo bidimensionnelle (12) couplée au convertisseur vidéo 3D (14), dans lequel la source vidéo bidimensionnelle fournit le signal vidéo bidimensionnel.

7. Système selon la revendication 6, dans lequel les signaux de stimulation tridimensionnels fournissent une stimulation représentative de l'accélération dans chacune des directions de tangage, de lacet et de roulis.
